(19) Europäisches Patentamt
European Patent Office
Office européen des brevets

(11) Veröffentlichungsnummer: **0 414 216 A2**

# (12) EUROPÄISCHE PATENTANMELDUNG

(21) Anmeldenummer: **90116039.0**

(22) Anmeldetag: **22.08.90**

(51) Int. Cl.5: **C07D 213/81**, A61K 31/44

(30) Priorität: **25.08.89 DE 3928144**

(43) Veröffentlichungstag der Anmeldung:
**27.02.91 Patentblatt 91/09**

(84) Benannte Vertragsstaaten:
**AT BE CH DE DK ES FR GB GR IT LI LU NL SE**

(71) Anmelder: **HOECHST AKTIENGESELLSCHAFT**
**Postfach 80 03 20**
**D-6230 Frankfurt am Main 80(DE)**

(72) Erfinder: **Baader, Ekkehard, Dr.**
**Amselweg 14**
**D-6240 Königstein/Taunus(DE)**
Erfinder: **Bickel, Martin, Dr.**
**Mittelstedter Weg 3**
**D-6380 Bad Homburg(DE)**
Erfinder: **Günzler-Pukall, Volkmar, Dr.**
**Gross-Seelheimer Strasse 13**
**D-3550 Marburg(DE)**

## (54) Zyklische Pyridin-2,4-und-2,5-dicarbonsäurediamide, Verfahren zu deren Herstellung sowie deren Verwendung.

(57) Die Erfindung betrifft zyklische Pyridin-2,4- und -2,5-dicarbonsäurediamide der Formel I

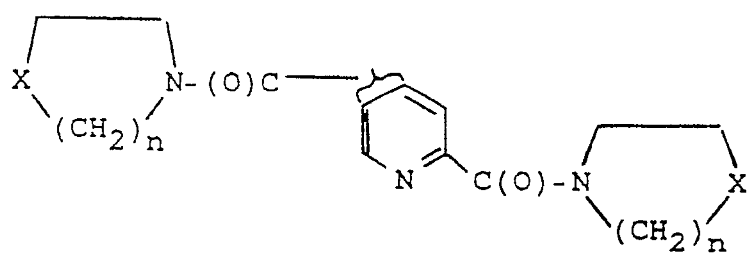

(I)

worin n und X die angegebenen Bedeutungen haben. Die erfindungsgemäßen Verbindungen hemmen die Prolin- und Lysinhydroxylase und können dementsprechend als Fibrosuppressiva und Immunsuppressiva eingesetzt werden.

EP 0 414 216 A2

# ZYKLISCHE PYRIDIN-2,4- UND -2,5-DICARBONSÄUREDIAMIDE, VERFAHREN ZU DEREN HERSTELLUNG SOWIE DEREN VERWENDUNG

Verbindungen, die die Prolin- und Lysinhydroxylase inhibieren, bewirken eine sehr selektive Hemmung der Kollagenbiosynthese durch Beeinflussung der kollagenspezifischen Hydroxylierungsreaktionen. In deren Verlauf wird Protein-gebundenes Prolin oder Lysin durch die Enzyme Prolin- bzw. Lysinhydroxylase hydroxyliert. Wird diese Reaktion durch Inhibitoren unterbunden, so entsteht ein nicht funktionsfähiges, unterhydroxyliertes Kollagenmolekül, das von den Zellen nur in geringer Menge in den extrazellulären Raum abgegeben werden kann. Das unterhydroxylierte Kollagen kann außerdem nicht in die Kollagenmatrix eingebaut werden und wird sehr leicht proteolytisch abgebaut. Als Folge dieser Effekte verringert sich insgesamt die Menge des extrazellulär abgelagerten Kollagens.

Es ist bekannt, daß die Inhibierung der Prolinhydroxylase durch bekannte Inhibitoren, wie $\alpha,\alpha'$-Dipyridyl zu einer Hemmung der $C1_q$-Biosynthese von Makrophagen führt (W. Müller et al., FEBS Lett. 90 (1978), 218; Immunbiology 155 (1978) 47). Dadurch kommt es zu einem Ausfall des klassischen Weges der Komplementaktivierung. Inhibitoren der Prolinhydroxylase wirken daher auch als Immunsuppressiva, z.B. bei Immunkomplexkrankheiten.

Es ist bekannt, daß Prolinhydroxylase durch Pyridin-2,4-und -2,5-dicarbonsäure effektiv gehemmt wird (K. Majamaa et al., Eur. J. Biochem. 138 (1984) 239-245). Diese Verbindungen sind in der Zellkultur allerdings nur in sehr hohen Konzentrationen als Hemmstoffe wirksam (Tschank, G. et al., Biochem. J. 238, 625-633, 1987).

In der DE-A 34 32 094 werden Pyridin-2,4- und -2,5-dicarbonsäurediester mit 1-6 C-Atomen im Esteralkylteil als Arzneimittel zur Inhibierung der Prolin-und Lysinhydroxylase beschrieben.

Diese niedrig-alkylierten Diester haben jedoch den Nachteil, daß sie zu schnell im Organismus zu den Säuren gespalten werden und nicht in genügend hoher Konzentration an ihren Wirkort in der Zelle gelangen und damit für eine eventuelle Verabreichung als Arzneimittel weniger geeignet sind.

Die DE-A 37 03 959, DE-A 37 03 962 und DE-A 37 03 963 beschreiben in allgemeiner Form gemischte Ester/Amide, höher alkylierte Diester und Diamide der Pyridin-2,4-und-2,5-dicarbonsäure, die die Kollagenbiosynthese im Tiermodell wirksam hemmen.

So wird in der DE-A 37 03 959 unter anderem die Synthese von N,N'-Bis(2 methoxyethyl)-pyridin-2,4-dicarbonsäurediamid und N,N'-Bis(3-isopropoxypropyl)-pyridin-2,4-dicarbonsäurediamid beschrieben.

In den deutschen Patentanmeldungen P 38 26 471.4 und P 38 28 140.6 wird ein verbessertes Verfahren zur Herstellung von N,N'-Bis(2-methoxyethyl)-pyridin-2,4-dicarbonsäurediamid vorgeschlagen. Die deutsche Patentanmeldung P 3924093.2 (HOE 89/F 241) schlägt neue N,N'-Bis(alkoxy-alkyl)-pyridin-2,4-dicarbonsäurediamide vor.

Überraschend wurde nun gefunden, daß zyklische Pyridin-2,4- und -2,5-dicarbonsäurediamide der Formel I

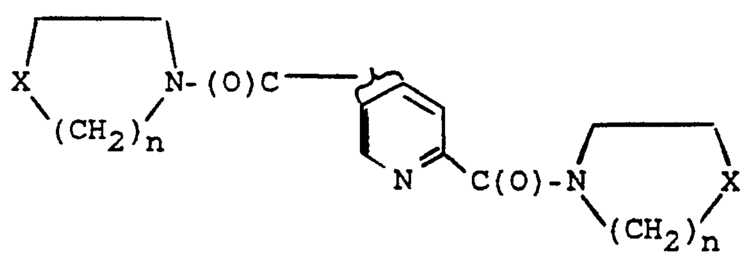

(I)

worin

n 1 bis 3 bedeutet und

X 0, S oder N-$R^1$ bedeutet,

wobei

$R^1$ verzweigtes oder unverzweigtes $C_1$-$C_6$-Alkyl, $C_1$-$C_6$ Alkenyl oder $C_1$-$C_6$-Alkinyl bedeutet, wobei diese Alkyl-, Alkenyl- und Alkinylreste unsubstituiert sind oder ein- oder mehrfach substituiert sind mit:

Phenyl, welches seinerseits ein- oder mehrfach substituiert ist mit einem oder mehreren Substituenten, ausgewählt aus:

Halogen, Nitro, Cyano, Carboxy, Hydroxy, Methyl, Ethyl, Methoxy, Ethoxy und Trifluormethyl,

oder

$N(R^2)_2$, wobei
$R^2$ H oder $C_1$-$C_3$-Alkyl bedeutet,
oder
$COOR^3$, wobei
$R^3$ H oder $C_1$-$C_3$-Alkyl bedeutet,
oder
$CON(R^4)_2$, wobei
$R^4$ H oder $C_1$-$C_3$-Alkyl bedeutet, oder wobei $(R^4)_2$ eine $C_4$-$C_6$-Alkylenkette darstellt, worin keine oder eine $CH_2$-Gruppe, welche nicht direkt benachbart zu dem Stickstoffatom steht, ersetzt ist durch 0, S oder N-$R^2$
oder wobei
$R^1$ $C_1$-$C_4$-Alkoxy-carbonyl oder $C_3$-$C_7$-Cycloalkyl bedeutet
sowie die physiologisch verträglichen Salze,
ebenfalls die Lysin- und Prolin-hydroxylase im Tiermodell wirksam inhibieren. Überraschend wurde hierbei ebenfalls gefunden, daß die erfindungsgemäßen Verbindungen im Vergleich zu den in DE-A 3 703 959, DE-A 3 703 962 und DE-A 3 703 963 beschriebenen Verbindungen eine deutlich bessere Resorbierbarkeit aufweisen.

Unter Halogen werden Fluor, Chlor, Brom und Jod verstanden.

"Mehrfach substituiert" bedeutet im Vorstehenden und Folgenden, daß mindestens 2, höchstens alle in den Alkyl-, Alkenyl-, Alkinyl- und Phenylresten vorhandenen Wasserstoffatome durch die genannten Substituenten ersetzt sind. Bei Mehrfachsubstitutionen können die Substituenten auch voneinander unabhängig verschieden sein.

Weiterhin betrifft die Erfindung ein Verfahren zur Herstellung von Verbindungen der Formel I, das dadurch gekennzeichnet ist, daß man
eine Verbindung der Formel II

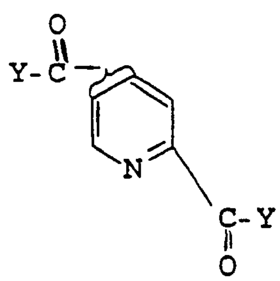

(II)

worin
Y Halogen oder Hydroxy ist oder zusammen mit der Cabonylgruppe einen Aktivester oder ein gemischtes Anhydrid bildet,
mit einer Verbindung der Formel III

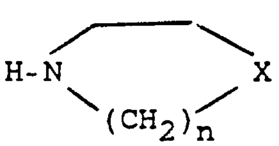

(III)

worin n und X die oben zu Formel I angegebenen Bedeutungen haben,
umsetzt und die Reaktionsprodukte gegebenenfalls in ihre physiologisch verträglichen Salze überführt.

Im folgenden wird die Herstellung von Verbindungen gemäß Formel I und die Herstellung der dafür benötigten Ausgangssubstanzen - sofern sie nicht käuflich sind -näher beschrieben.

Die Herstellung der erfindungsgemäßen Verbindungen gelingt am einfachsten dadurch, daß die beiden Komponenten, das Pyridin-Derivat gemäß Formel (II) und das Amin gemäß Formel (III) in äquimolaren Mengen oder bis zu einem etwa 5-fachen Überschuß an III, vermengt werden und bei Temperaturen zwischen -30 bis 150 °C, bevorzugt bei 20 bis 100 °C bis zur Beendigung der Reaktion umgesetzt werden. Die Beendigung der Reaktion läßt sich mittels Dünnschichtchromatographie (DC-Kontrolle) bestimmen. Eine Variante dieses Verfahrens besteht darin, daß man in einem geeigneten Lösungsmittel, wie Diäthyläther oder Dimethoxyäthan oder Tetrahydrofuran, chlorierten Kohlenwasserstoffen wie Methylenchlorid, Chloroform, Tri- oder Tetrachloräthylen, Benzol, Toluol oder auch polaren Lösungsmitteln wie Dimethylformamid oder Aceton oder Dimethylsulfoxid arbeitet. Auch hier kann ein Überschuß von Amin gemäß Formel (III), der bis zur etwa 5-fachen Menge betragen kann, angewandt werden. Die Reaktionstemperaturen liegen dabei zwischen Raumtemperatur und dem Siedepunkt des Lösungsmittels, wobei Temperaturen im Bereich von Raumtemperatur bis 130 ° C besonders bevorzugt sind.

Ebenso kann die Umsetzung über ein gemischtes Anhydrid wie Chlorameisensäureethylester oder über einen aktivierten Ester wie Paranitrophenylester (Y = $ClCH_2$-COO oder $NO_2$-$C_6H_4$-O) erfolgen. Entsprechende Methoden sind in der Literatur beschrieben.

Gegebenenfalls kann die Umsetzung auch in Gegenwart von Basen erfolgen. Als zusätzliche Basen kommen anorganische Säurefänger wie Carbonate oder Hydrogencarbonate z.B. Natrium- oder Kaliumcarbonat oder Natrium- oder Kaliumhydrogencarbonat, oder organische Säurefänger wie tertiäre Amine, wie Triäthylamin, Tributylamin, Äthyldiisopropylamin oder heterocyclische Amine wie N-Alkylmorpholin, Pyridin, Chinolin oder Dialkylaniline in Betracht.

Bevorzugt erfolgt die Umsetzung der Verbindungen gemäß Formel (II) mit Aminen gemäß Formel (III) unter Zusatz eines wasserabspaltenden Mittels wie Dialkylcarbodiimid, wobei die Alkylreste 1 bis 8 C-Atome aufweisen, die im Falle der $C_3$-$C_8$-Verbindungen auch verzweigt oder cyclisch sein können; bevorzugt wird Dicyclohexylcarbodiimid eingesetzt. Eine entsprechende Methode ist in der Literatur beschrieben.

Gegebenenfalls kann die Aufarbeitung der Produkte beispielsweise durch Extraktion oder durch Chromatographie z.B. über Kieselgel erfolgen. Das isolierte Produkte kann umkristallisiert und gegebenenfalls mit einer geeigneten Säure zu einem physiologisch verträglichen Salz umgesetzt werden. Als geeignete Säuren kommen beispielsweise in Betracht:
Mineralsäuren, wie Chlorwasserstoff- und Bromwasserstoffsäure sowie Schwefel-, Phosphor-, Salpeter-oder Perchlorsäure oder organische Säuren wie Ameisen-, Essig-, Propion-, Bernstein-, Glykol-, Milch-, Äpfel-, Wein-, Zitronen-, Malein-, Fumar-, Phenylessig-, Benzoe-, Methansulfon-, Toluolsulfon-, Oxal-, 4-Aminobenzoe-, Naphthalin-1,5-disulfon- oder Ascorbinsäure.

Die Ausgangsverbindungen der Formel (III) können, soweit sie nicht käuflich sind, einfach synthetisiert werden (z.B. Organikum, Organisch Chemisches Grundpraktikum, 15. Aufl., VEB Deutscher Verlag der Wissenschaften, 1976; eine Übersicht über die verschiedenen Möglichkeiten findet sich im Methodenregister, S. 822).

Die Ausgangsverbindungen der Formel (II) erhält man beispielsweise durch Umsetzung von Pyridin-2,4- oder -2,5-dicarbonsäure zu dem entsprechenden Pyridin-2,4- oder -2,5-dicarbonsäurehalogenid, bevorzugt -chlorid (nach literaturbekannten Verfahren), welches dann mit einem geeigneten Alkohol, z.B. Paranitrobenzylalkohol zu dem entsprechenden Aktivester umgesetzt wird. Ebenso kann die Pyridin-2,4- oder -2,5 dicarbonsäure auch zunächst unter Zusatz einer geeigneten Carbonsäure oder eines Carbonsäureesters wie Chlorameisensäureethylester in ein gemischtes Anhydrid überführt werden, welches dann mit den Aminen (III) zu den erfindungsgemäßen Produkten umgesetzt wird. Eine entsprechende Methode ist in der Literatur beschrieben.

Die erfindungsgemäßen Verbindungen der Formel I besitzen wertvolle pharmakologische Eigenschaften und zeigen insbesondere Wirksamkeit als Hemmer der Prolin- und Lysinhydroxylase, als Fibrosuppressivum und Immunsuppressivum.

Die Aktivität der Fibrogenase kann durch radioimmunologische Bestimmung des N-terminalen Propeptids des Kollagens TYP-III oder der N- bzw. C terminalen Quervernetzungsdomäne des Kollagens-Typ-IV (7s-Kollagen bzw. Typ-IV-Kollagen-$NC_1$) im Serum bestimmt werden.

Zu diesem Zweck wurden die Hydroxyprolin-, Prokollagen-III-Peptid-, 7s-Kollagen- und Typ-IV-Kollagen-$NC_1$-Konzentrationen in der Leber von

a) unbehandelten Ratten (Kontrolle)

b) Ratten, denen Tetrachlorkohlenstoff verabreicht wurde ($CCl_4$-Kontrolle)

c) Ratten, denen zunächst $CCl_4$ und anschließend eine erfindungsgemäße Verbindung verabreicht wurde gemessen (diese Testmethode wird beschrieben von Rouiller, C., experimental toxic injury of the liver; in The Liver, C. Rouiller, Vol. 2, S. 335-476, New York, Academic Press, 1964).

Die Verbindungen der Formel I können als Medikamente in Form pharmazeutischer Präparate Verwendung finden, welche sie gegebenenfalls zusammen mit verträglichen pharmazeutischen Trägern enthalten. Die Verbindungen können als Heilmittel, z.B. in Form pharmazeutischer Präparate Verwendung finden, welche diese Verbindungen in Mischung mit einem für die enterale, perkutane oder parenterale Applikation geeigneten pharmazeutischen, organischen oder anorganischen Träger, wie z.B. Wasser, Gummi arabicum, Gelatine, Milchzucker, Stärke, Magnesiumstearat, Talk, pflanzliche Öle, Polyalkylenglykole, Vaseline usw. enthalten.

Die pharmazeutischen Präparate können in fester Form, z.B. als Tabletten, Dragees, Suppositorien oder

Kapseln; in halbfester Form, z.B. als Salben, oder in flüssiger Form, z.B. als Lösungen, Suspensionen oder Emulsionen, vorliegen. Gegebenenfalls sind sie sterilisiert und/oder enthalten Hilfsstoffe, wie Konservierungs-, Stabilisierungs-, Netz oder Emulgiermittel, Salze zur Veränderung des osmotischen Drucks oder Puffer. Sie können auch noch andere therapeutisch wirksame Stoffe enthalten.

Im folgenden wird die Erfindung anhand von Beispielen näher erläutert:

**Beispiel 1**

**2,4-Di-[(thiomorpholin-1-yl)-carbonyl]-pyridin**

5 g (0,03 mol) Pyridin-2,4-dicarbonsäure werden in 100 ml Toluol suspendiert. Bei Raumtemperatur werden 4,4 ml (0,06 mol) Thionylchlorid + 1 ml Dimethylformamid zugetropft. Es wird für 2 Stunden unter Rückfluß gekocht, bis die entstandene Gasentwicklung beendet ist und die Lösung klar geworden ist. Es wird auf 0°C abgekühlt und eine Lösung von 60 ml (0,06 mol) Thiomorpholin und 10,4 ml (0,075 mol) Triethylamin in 20 ml Toluol zugetropft.

Es wird 12 Stunden bei Raumtemperatur gerührt, einmal mit gesättigter Natriumhydrogencarbonat-Lösung gewaschen. Die wäßrige Phase wird noch zweimal mit Toluol ausgeschüttelt, die vereinigten organischen Phasen über Magnesiumsulfat getrocknet und eingedampft. Der Rückstand wird mit Diethylether verrieben und abgesaugt.

Ausbeute: 5,2 g Schmelzpunkt: 117 - 119°C

$^1$H-NMR : $\delta$ = 2,47 - 2,93 (m, 8H); 3,53 - 4,15 (m, 8H); 7,25 (m, 1H); 7,60 (m, 1H); 8,70 (m, 1H);

**Beispiel 2**

**2,4-Di-[(morpholin-1-yl)-carbonyl]-pyridin**

Analog zu Beispiel 1 wird das Säurechlorid hergestellt und mit 5,3 ml (0,06 mol) Morpholin umgesetzt.

Ausbeute: 6,7 g Schmelzpunkt: 126 - 127°C

$^1$H-NMR : $\delta$ = 3,50 - 4,00 (m, 16H); 7,30 (m, 1H); 7,70 (m, 1H); 8,70 (m, 1H)

**Beispiel 3**

**2,4-Di-[(1-methylpiperazin-4-yl)-carbonyl]-pyridin**

Analog zu Beispiel 1 wird das Säurechlorid hergestellt und mit 6,7 ml (0,06 mol) N-methylpiperazin umgesetzt.

Ausbeute 6,7 g Schmelzpunkt 125°C

$^1$H NMR: = 2,30 (s, 6H); 2,40 (m, 4H); 2,5 (m, 4H); 3,40 (m, 2H); 3,60 (m, 2H); 3,80 (m, 4H); 7,30 (m, 1H); 7,60 (m, 1H); 8,65 (m, 1H)

**Ansprüche**

1. Cyclische Pyridin-2,4- und -2,5-dicarbonsäurediamide der Formel I

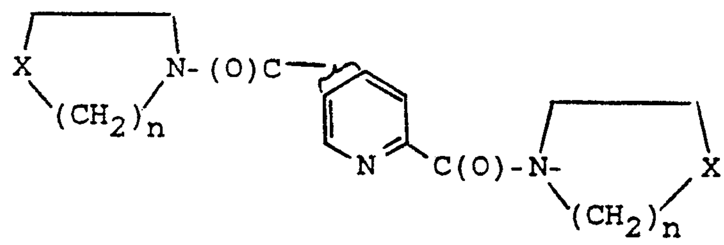

(I)

worin

n 1 bis 3 bedeutet und

X 0,S oder N-$R^1$ bedeutet,

wobei

$R^1$ verzweigtes oder unverzweigtes $C_1$-$C_6$-Alkyl, $C_1$-$C_6$-Alkenyl oder $C_1$-$C_6$-Alkinyl bedeutet, wobei diese Alkyl-, Alkenyl- und Alkinylreste unsubstituiert sind oder ein- oder mehrfach substituiert sind mit:

Phenyl, welches seinerseits ein- oder mehrfach substituiert ist mit einem oder mehreren Substituenten, ausgewählt aus:

Halogen, Nitro, Cyano, Carboxy, Hydroxy, Methyl, Ethyl, Methoxy, Ethoxy und Trifluormethyl,

oder

$N(R^2)_2$, wobei

$R^2$ H oder $C_1$-$C_3$-Alkyl bedeutet,

oder

$COOR^3$, wobei

$R^3$ H oder $C_1$-$C_3$-Alkyl bedeutet,

oder

$CON(R^4)_2$, wobei

$R^4$ H oder $C_1$-$C_3$-Alkyl bedeutet, oder wobei $(R^4)_2$ eine $C_4$-$C_6$-Alkylenkette darstellt, worin keine oder eine $CH_2$-Gruppe, welche nicht direkt benachbart zu dem Stickstoffatom steht, ersetzt ist durch 0, S oder N-$R^2$

oder wobei

$R^1$ $C_1$-$C_4$-Alkoxy-carbonyl oder $C_3$-$C_7$-Cycloalkyl bedeutet

sowie die physiologisch verträglichen Salze.

2. Cyclische Pyridin-2,4- und -2,5-dicarbonsäurediamide gemäß Formel I nach Anspruch 1, worin mindestens eine der nachfolgenden Bedingungen erfüllt ist:

$R^1$ bedeutet

verzweigtes oder unverzweigtes $C_1$-$C_6$-Alkyl oder $C_1$-$C_6$-Alkenyl, wobei diese Alkyl- und Alkenylreste unsubstituiert sind oder ein- oder mehrfach substituiert sind mit

Phenyl, welches seinerseits ein- oder mehrfach substituiert ist mit einem oder mehreren Substituenten, ausgewählt aus:

Fluor, Chlor, Methyl, Ethyl, Methoxy, Ethoxy und Trifluormethyl

$R^2$ bedeutet H oder $C_1$-$C_2$-Alkyl

$R^3$ bedeutet H oder $C_1$-$C_2$-Alkyl

$R^4$ bedeutet H oder $C_1$-$C_2$-Alkyl oder

$(R^4)_2$ bedeutet eine $C_4$-$C_5$-Alkylenkette worin keine oder eine $CH_2$-Gruppe, welche nicht direkt benachbart zu dem Stickstoffatom steht, ersetzt ist durch 0,S oder N-$R^2$.

3. Cyclische Pyrridin-2,4- und -2,5-dicarbonsäurediamide gemäß Formel I nach Anspruch 1 oder 2, worin mindestens eine der nachfolgenden Bedingungen erfüllt ist:

$R^1$ bedeutet verzweigtes oder unverzweigtes $C_1$-$C_6$-Alkyl, $C_1$-$C_4$-Alkoxy-carbonyl, oder $C_3$-$C_7$-Cycloalkoxy.

4. Cyclische Pyridin-2,4- und -2,5-dicarbonsäurediamide gemäß Formel I nach einem oder mehreren der Ansprüche 1 -3, worin mindestens eine der nachfolgenden Bedingungen erfüllt ist:

n bedeutet 2

X bedeutet 0 oder S.

5. Verfahren zur Herstellung von Cyclischen Pyridin-2,4-und -2,5-dicarbonsäurediamiden der Formel I nach Anspruch 1, dadurch gekennzeichnet, daß man eine Verbindung der Formel II

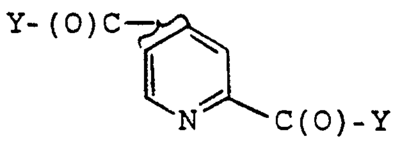

(II)

worin

Y Halogen oder Hydroxy ist oder zusammen mit der Cabonylgruppe einen Aktivester oder ein gemischtes Anhydrid bildet,

mit einer Verbindung der Formel III

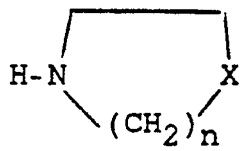

(III)

worin n und X die in Anspruch 1 zu Formel I angegebenen Bedeutungen haben,

umsetzt und die Reaktionsprodukte gegebenenfalls in ihre physiologisch verträglichen Salze überführt.

6. Verfahren nach Anspruch 5, dadurch gekennzeichnet, daß die Umsetzung unter gleichzeitiger Hinzugabe von Dialkylcarbodiimid erfolgt, wobei die Dialkylreste 1 bis 8 C-Atome aufweisen, die im Falle der $C_3$-$C_8$-Verbindungen auch verzweigt oder cyclisch sein können.

7. Verbindungen nach einem oder mehreren der Ansprüche 1 bis 4 zur Inhibierung der Prolin- und Lysinhydroxylase.

8. Verbindungen nach einem oder mehreren der Ansprüche 1 bis 4 zur Anwendung als Fibrosuppressiva und Immunsuppressiva.

9. Arzneimittel, enthaltend eine Verbindung der Formel I mit verträglichen pharmazeutischen Trägern.

10. Verwendung von Verbindungen der Formel I zur Beeinflussung des Stoffwechsels von Collagen und collagenähnlichen Stoffen bzw. der Biosynthese von $C1_q$.

11. Verwendung von Verbindungen der Formel I zur Behandlung von Störungen des Stoffwechsels von Collagen und collagenähnlichen Stoffen bzw. der Biosynthese von $C1_q$.

12. Verfahren zur Herstellung von Arzneimitteln zur Beeinflussung des Stoffwechsels von Collagen und collagenähnlichen Stoffen bzw. der Biosynthese von $C1_q$, dadurch gekennzeichnet, daß man in das Arzneimittel eine Verbindung der Formel I einverleibt.

Patentansprüche für folgende Vertragsstaaten:ES,GR

1. Verfahren zur Herstellung von cyclischen Pyridin-2,4- und -2,5-dicarbonsäurediamiden der Formel (I)

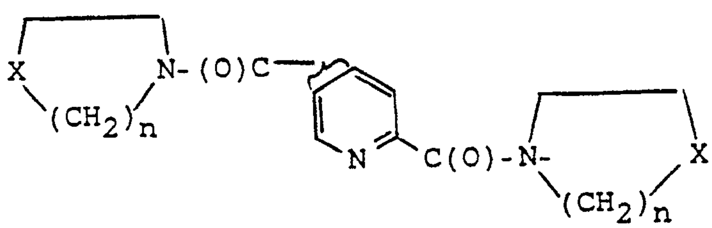

(I)

worin

n 1 bis 3 bedeutet und

X O,S oder N-$R^1$ bedeutet,

wobei

$R^1$ verzweigtes oder unverzweigtes $C_1$-$C_6$-Alkyl, $C_1$-$C_6$-Alkenyl oder $C_1$-$C_6$-Alkinyl bedeutet, wobei diese Alkyl-, Alkenyl- und Alkinylreste unsubstituiert sind oder ein-oder mehrfach substituiert sind mit:

Phenyl, welches seinerseits ein oder mehrfach substituiert ist mit einem oder mehreren Substituenten, ausgewählt aus:

Halogen, Nitro, Cyano, Carboxy, Hydroxy, Methyl, Ethyl, Methoxy, Ethoxy und Trifluormethyl,

oder
$N(R^2)_2$, wobei
$R^2$ H oder $C_1$-$C_3$-Alkyl bedeutet,
oder
$COOR^3$, wobei
$R^3$ H oder $C_1$-$C_3$-Alkyl bedeutet,
oder
$CON(R^4)_2$, wobei
$R^4$ H oder $C_1$-$C_3$-Alkyl bedeutet, oder wobei $(R^4)_2$ eine $C_4$-$C_8$-Alkylenkette darstellt, worin keine oder eine $CH_2$-Gruppe, welche nicht direkt benachbart zu dem Stickstoffatom steht, ersetzt ist durch O, S oder N-$R^2$
oder wobei
$R^1$ $C_1$-$C_4$-Alkoxy-carbonyl oder $C_3$-$C_7$-Cycloalkyl bedeutet
sowie die physiologisch verträglichen Salze, dadurch gekennzeichnet, daß man eine Verbindung der Formel II

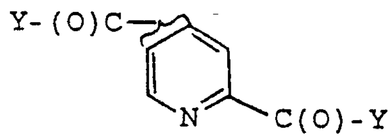

(II)

worin
Y Halogen oder Hydroxy ist oder zusammen mit der Cabonylgruppe einen Aktivester oder ein gemischtes Anhydrid bildet,
mit einer Verbindung der Formel III

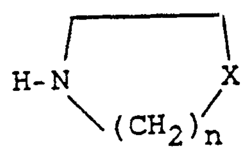

(III)

worin n und X die in Anspruch 1 zu Formel 2 angegebenen Bedeutungen haben,
umsetzt und die Reaktionsprodukte gegebenenfalls in ihre physiologisch verträglichen Salze überführt.

2. Verfahren zur Herstellung von cyclischen Pyridin-2,4- und -2,5-dicarbonsäurediamiden gemäß Formel I nach Anspruch 1, worin mindestens eine der nachfolgenden Bedingungen erfüllt ist:
$R^1$ bedeutet
verzweigtes oder unverzweigtes $C_1$-$C_6$-Alkyl oder $C_1$-$C_6$-Alkenyl, wobei diese Alkyl-und Alkenylreste unsubstituiert sind oder ein- oder mehrfach substituiert sind mit:
Phenyl, welches seinerseits ein- oder mehrfach substituiert ist mit einem oder mehreren Substituenten, ausgewählt aus:
Fluor, Chlor, Methyl, Ethyl, Methoxy, Ethoxy und Trifluormethyl
$R_2$ bedeutet H oder $C_1$-$C_2$-Alkyl
$R_3$ bedeutet H oder $C_1$-$C_2$-Alkyl
$R^4$ bedeutet H oder $C_1$-$C_2$-Alkyl oder
$(R^4)_2$ bedeutet eine $C_4$-$C_5$-Alkylenkette, worin keine oder eine $CH_2$-Cruppe, welche nicht direkt benachbart zu dem Stickstoffatom steht, ersetzt ist durch 0,S oder N-$R^2$.

3. Verfahren zur Herstellung von cyclischen Pyridin-2,4- und -2,5-dicarbonsäurediamiden gemäß Formel I nach Anspruch 1 oder 2, worin mindestens eine der nachfolgenden Bedingungen erfüllt ist:
$R^1$ bedeutet verzweigtes oder unverzweigtes $C_1$-$C_6$-Alkyl, $C_1$-$C_4$-Alkoxycarbonyl, oder $C_3$-$C_7$-Cycloalkoxy.

4. Verfahren zur Herstellung von cyclischen Pyridin-2,4- und -2,5-dicarbonsäurediamiden gemäß Formel I nach einem oder mehreren der Ansprüche 1-3, worin mindestens eine der nachfolgenden Bedingungen erfüllt ist:
n bedeutet 2
X bedeutet 0 oder S.

5. Verfahren nach Anspruch 4, dadurch gekennzeichnet, daß die Umsetzung unter gleichzeitiger Hinzugabe von Dialkylcarbodiimid erfolgt, wobei die Dialkylreste 1 bis 8 C-Atome aufweisen, die im Falle der $C_3$-$C_8$-Verbindungen auch verzweigt oder cyclisch sein können.

6. Verbindungen nach einem oder mehreren der Ansprüche 1 bis 5 zur Inhibierung der Prolin- und Lysinhydroxylase.

7. Verbindungen nach einem oder mehreren der Ansprüche 1 bis 5 zur Anwendung als Fibrosuppressiva und Immunsuppressiva.

8. Arzneimittel, enthaltend eine Verbindung der Formel 2 mit verträglichen pharmazeutischen Trägern.

9. Verwendung von Verbindungen der Formel 2 zur Beeinflussung des Stoffwechsels von Collagen und collagenähnlichen Stoffen bzw. der Biosynthese von $Cl_q$.

10. Verwendung von Verbindungen der Formel 2 zur Behandlung von Störungen des Stoffwechsels von Collagen und collagenähnlichen Stoffen bzw. der Biosynthese von $Cl_q$.

11. Verfahren zur Herstellung von Arzneimitteln zur Beeinflussung des Stoffwechsels von Collagen und collagenähnlichen Stoffen bzw. der Biosynthese von $Cl_q$, dadurch gekennzeichnet, daß man in das Arzneimittel eine Verbindung der Formel 2 einverleibt.